# EUROPEAN PATENT SPECIFICATION

(11) **EP 1 079 798 B1**
(45) Date of publication and mention of the grant of the patent: **16.07.2003**
(21) Application number: 99926402.1
(22) Date of filing: 21.05.1999
(51) Int. Cl.: A61K 7/48, A61K 7/36, A61K 33/30, A61K 7/16

(54) **ODOUR ABSORBING AGENT**
GERUCHSABSORBER
AGENT ABSORBANT LES ODEURS

(30) Priority: 21.05.1998 GB 9810806
(43) Date of publication of application: 07.03.2001
(73) Proprietor: The Boots Company PLC, Nottingham, Nottinghamshire NG2 3AA (GB)
(72) Inventor: BUTCHER, Kate Elizabeth, Nottingham NG2 3AA (GB); DE GRAAF, Thalie Paulina, Nottingham NG2 3AA (GB); GALLEY, Edward, Nottingham NG2 3AA (GB)
(74) Representative: Jones, Stephen Anthony
(86) International application number: EP9903591
(87) International publication number: WO99059539

(56) References cited:
- EP-A- 0 564 307
- WO-A-94/14406
- WO-A-95/13806
- WO-A-98/11867
- GB-A- 2 233 227
- US-A- 4 239 781
- PATENT ABSTRACTS OF JAPAN vol. 1996, no. - , 26 December 1996 (1996-12-26) & JP 08 217637 A (KAO CORP), 27 August 1996 (1996-08-27)
- PATENT ABSTRACTS OF JAPAN vol. 1999, no. 053, 31 May 1999 (1999-05-31) & JP 11 049637 A (KAO CORP), 23 February 1999 (1999-02-23)
- PATENT ABSTRACTS OF JAPAN vol. 1996, no. - , 29 February 1996 (1996-02-29) & JP 07 277914 A (KAO CORP), 24 October 1995 (1995-10-24)

## Description

This invention teaches the use of a new form of zinc oxide as an agent to absorb unpleasant odours.

The human sense of smell is acute. Some of the most embarrassing human conditions are related to an unpleasant smell such as bad breath and body odour. In order to prevent such socially unacceptable situations, there are a multitude of compositions available, which aim to prevent odour by masking the noxious smells. This does not remove the odour, just covers it with a stronger, more pleasant odour. There are other compositions which try and stop something starting to smell.

Further, there are many compositions designed for use on humans, in particular for application to the skin or hair, whose odour is most unpleasant. If it is not possible to mask such smells, then there is no other option but to tolerate a foul smell when using such a product.

There are certain criteria that any agent used to prevent, absorb or mask odour should meet. They should be effective and cosmetically acceptable. When incorporated into compositions for use they should possess a pleasant feel, or taste and should not cause staining. There are no agents used at present which conform to all these exacting standards. One of the most successful biocidals used in oral hygiene compositions is chlorhexidine which kills the bacteria which produce the volatile sulphur compounds responsible for bad breath. Its disadvantages are an unpleasant taste, and a tendency to stain the teeth brown. Clearly there is a need for a new agent capable of preventing, absorbing or masking odour.

This invention discloses the new use of a specific form of zinc oxide which has vastly superior odour absorbence properties, without many of the disadvantages of conventional odour masking agents. This material has an extremely high surface area, between 30m²/g and 100m²/g, most preferably above 90m²/g. The zinc oxide particles have a diameter between 0.01 and 200µm. Hereinafter, such zinc oxide particles shall be known as "high surface area zinc oxide". The surface area of the material can be determined using laser diffraction. From the surface area, the mean estimated spherical diameter may be calculated. The diameter of the particles is henceforth taken to mean the mean estimated spherical diameter. The surface area of the high surface area zinc oxide particles used with this invention was calculated using a Micromeritics Flavsorb II 2300 BET apparatus. A suitable zinc oxide is commercially available as Activox C80 from Elementis.

The production of such zinc oxides is well documented.

### WO 95/04704 (Harcros)

This patent discloses a method for the production of zinc oxide in the form of discrete particles which have an average particle size of 0.08µm or less in diameter and a surface area >12.5m²/g. The zinc oxide produced is found to be particularly good as an additive for scattering/absorbing UV light.

### S. Tichy, SOFW - Journal 119 Jahrgang 8/93

This article teaches a method for producing zinc oxide with a particle size of 0.2µm and a surface area of 50-150m²/g.

### Liu et al - Journal of Materials Science 21 (1986) 3698-3702

This describes another method of producing zinc oxide particles with a diameter of 0.15µm and a surface area of about 50m²/g.

These articles are but a small selection from a large number of articles detailing the manufacture of this particular type of zinc oxide particles.

The present invention provides the use of high surface area zinc oxide powder having a surface area of between 30 m²/g and 100 m²/g and a particle diameter between 0.1 and 200 µm and an acceptable diluent or carrier in the preparation of an odour-absorbing composition in which the high surface area zinc oxide absorbs the odour.

Bad breath can be a highly embarrassing and socially unacceptable condition. It may be caused by bad teeth, infections of the gums, indigestion or chronic tonsillitis. Diet also plays a role with certain foodstuffs, such as garlic giving a strong characteristic odour to the breath.

Remedies for this condition require care for the hygiene of the mouth. At one level this means dental treatment and care for the gums. There are also a number of more temporary solutions. Short term relief may be given by using a bacteriocides to kill the bacteria which breed in the mouth to produce odour. The odour may also be masked by a further odour such as mint. A further remedy is the use of an agent to absorb the noxious compounds which are present in bad breath.
Surprisingly, high surface area zinc oxide has been found to absorb the volatile sulfur compounds that are present in bad breath. Its pleasant mouthfeel, lack of unpleasant taste and its non-staining of teeth make it eminently suitable for incorporation into an odour absorbing oral composition.
Preferably the surface area of the high surface area zinc oxide is greater than 90 m²/g and the diameter of the particle is between 0.1 and 20.5 µm. In a preferred oral formulation the high surface area is present from 0.01 to 10% in the preparation of an odour absorbing composition for use in the oral cavity in which said high surface area zinc oxide absorbs the odour, preferably 0.1 to 5%, most preferably 0.3 to 1%, by weight of the total composition.

In a further embodiment of the invention, the composition for use in the oral cavity may be a toothpaste or a mouthwash. Such products may contain ingredients known to those skilled in the art. Such ingredients may include, but not limited to:-
a) non-cationic water-insoluble antibacterial agents such as bromochlorophene and triclosan;
b) cationic antibacterial agents such as cetylpyridinium chloride and chlorhexidine salts;
c) fluoride sources such as sodium fluoride, zinc fluoride, potassium, aluminium fluoride, lithium fluoride, sodium monofluorophosphates, acidulated phosphate fluoride, stannous fluoride, ammonium fluoride, ammonium bifluoride and amine fluoride;
d) abrasives such as silica, calcium carbonate, calcium phosphate, alumina and insoluble metaphosphates; synthetic resins, dicalcium phosphate, calcium pyrophosphate, natural and synthetic clays;
e) polyhydric alcohols such as xylitol, sorbitol, ethanol, glycerol, propylene glycol and polyethylene glycol;
f) preservatives such as methylparaben, sodium benzoate and other parabens;
g) surfactants such as sodium lauryl sulfate and including anionic, nonionic and amphoteric surfactants eg sodium methyl cocoyl taurate, sodium coconut monoglyceride sulphonates, alkyl aryl sulphonates and sodium salts, sodium salts of the coconut fatty acid amide of N-methyltaurine;
h) sweeteners such as sodium saccharin and aspartame;
i) antitartar agents such as metal ion citrates and alkali metal pyrophosphates;
j) thickeners such as cellulose gum, and other ingredients known to those skilled in the art;
k) pigment such as titanium dioxide and any other colour.

Further compositions for use in the oral cavity may include tablets to be taken orally, solid dosage forms intended to be sucked or masticated for example lozenges or chewing gum.
Solid compositions for oral administration may be the known pharmaceutical forms for such administration, for example tablets. Suitably tablets may be prepared by mixing the active components with an inert diluent in the presence of disintegrating agents, binding agents, flow aids and/or lubricating agents, and tableting the mixture by known methods. The tablets may be formulated in a manner known to those skilled in the art.

Suitable diluents include lactose, calcium phosphate, dextrin, microcrystalline cellulose, sucrose, starch, modified starch, calcium sulphate or mixtures thereof. Suitable lubricating agents include magnesium stearate, stearic acid, calcium stearate, sodium stearyl fumarate (sold under the trade name PRUV), hydrogenated vegetable oils or mixtures thereof. Preferably the lubricating agent is magnesium stearate or stearic acid. Suitable disintegrating agents include maize starch, sodium starch glycollate, low substituted hydroxypropyl cellulose, alginic acid, calcium carboxymethyl cellulose, croscarmellose or mixtures thereof. Suitable binders includes polyvinyl pyrrolidone, gelatin, hydroxypropyl methylcellulose, starch or mixtures thereof. Preferably the binder is polyvinyl pyrrolidone. Suitable flow aids include talc and colloidal silicon dioxide. It will be appreciated by those skilled in the art that a particular excipient may perform more than one function for example maize starch may act as a diluent, a binder or as a disintegrating agent.

Solid dosage forms intended to be sucked or masticated may be prepared by methods which are well known in the art for the production of lozenges or chewing gums and may contain other ingredients known in such dosage forms such as acidity regulators, opacifiers, stabilising agents, buffering agents, flavourings, sweeteners, colouring agents and preservatives. Lozenges may be prepared by heating the lozenge base (eg a mixture of sugar and liquid glucose) under vacuum to remove excess water and the remaining components are then blended into the mixture. The resulting mixture is then drawn into a continuous cylindrical mass from which the individual lozenges are formed. The lozenges are then cooled, and packed into suitable packaging eg a blister pack of a water-impermeable plastics material (eg polyvinylchloride) closed by a metallic eg aluminium foil. The user removes the lozenge by applying pressure to the blister to force the lozenge to rupture and pass through the metal foil seal. Lozenges will normally be sucked by the patient to release the components. Masticable solid dose formulations may be made by the methods used to prepare chewable candy products or chewing gums. For example, a chewable solid dosage form may be prepared from an extruded mixture of sugar and glucose syrup to which the high surface area zinc oxide has been added with optional addition of whipping agents, humectants, lubricants, flavours and colourings. (See Pharmaceutical Dosage Forms: Tablets, Volume 1, Second Edition edited by H A Lieberman, L Lachman and J B Schwartz published in 1989).

Such compositions for use in the oral cavity have been found to be highly efficacious in treating bad breath. Thus high surface area zinc oxide particles may be used in the preparation of a medicament for the treatment of bad breath. Such medicaments may be formulated as a mouthwash, a toothpaste, in tablet form or as chewing gum. Such products may be formulated in a manner known to those skilled in the art. Additional components, known to those skilled in the art may be included in the formulation.

Body odour can be a similarly embarrassing condition. An excess of sweat or sebum upon the skin is implicated as a factor in the causes of body odour. if not removed by washing, bacteria feed upon the sweat or sebum and produce an unpleasant odour. There are a multitude of compositions available associated with the condition, which work to mask, prevent or absorb the odour.

High surface area zinc oxide has been found to absorb the compounds responsible for body odour. As a mild non-irritating reagent, it may safely be incorporated into compositions to be applied to the human body. Thus, high surface area zinc oxide may be used to treat body odour.

Another embodiment of the present invention provides the use of high surface area zinc oxide having a surface area of between 30m²/g and 100m²/g and a particle diameter between 0.1 and 200 µm and a cosmetically acceptable diluent or carrier in the preparation of an odour absorbing composition formulated for topical application in the preparation of a topical odour absorbing composition in which said high surface area zinc oxide absorbs the odour.

Preferably, the surface area of the high surface area zinc oxide is greater than 90m²/g and the diameter of the particle is between 0.1 and 20.5µm.

In a preferred topical formulation the high surface area zinc oxide is present from 1-10%, preferably 3-8%, most preferably 4-6% by weight of the total composition.

With such topical compositions, the formulation may be a cosmetic composition suitable for application to human hair or skin. In a preferred embodiment the composition is used for the absorption of odour from the human body, such as deodorants or foot powder. Such products may be formulated in a manner known to those skilled in the art. Such products may contain ingredients commonly used by those skilled in the art. Such ingredients may include, though not limited to:-
a) antiperspirants such as aluminium chlorohydrate or aluminium zirconium;
b) deliquescents such as zea mays, rice starch, sodium bicarbonate, talc;
c) flow agents such as silica;
d) emulsifiers such as Steareth-2 and-21 and polypropylene glycol-15 stearyl ether.

There are a number of products designed for application to the human body whose odour is extremely strong and unpleasant. Conventional techniques such as odour masking generally have no effect upon the powerfully smelling components of products such as hairdyes, perming lotions and depilatories. Thus, there is no alternative but to tolerate an unpleasant smell when using such products.

It has been found that high surface area zinc oxide is capable of absorbing the noxious odours that such products produce. High surface area zinc oxide may be included into the formulation of a product and act to absorb the unpleasant odour of the composition without detracting from its efficacy.

A further embodiment of the invention provides the use of high surface area zinc oxide powder having a surface area of between 30 m²/g and 100 m²/g and a particle diametar of between 0.1 and 200 µm and and an unpleasant smelling ache in the preparation of a composition in which the high surface area zinc oxide has the effect of absorbing the smell of the unpleasant smelling active. Such unpleasant smelting actives may include, though not limited to:-
a) hair removing agents such as sodium, potassium or calcium thioglycolate;
b) skin tanning agents such as dihydroxyacetone;
c) ammonia in permanent hair dyes;
c) ammonium thioglycolate in hair perming products.

Suitable formulations may include, though not limited to hair dyes, hair perms, depilatories and fake tanning compositions. Such products may be formulated in a manner known to those skilled in the art. Such products may contain ingredients commonly used by those skilled in the art. Such ingredients may include though not limited to:-
a) antioxidants such as butylated hydroxytoluene, butylated hydroxyacetone or vitamin E;
b) preservatives such as methylparaben, sodium benzoate, any paraben;
c) thickeners such as salt, magnesium stearate or zinc stearate, xanthan gum, hydroxyethylcellulose and sodium magnesium silicate, polyethylene glycol-55 propylene, glycol oleate and propylene glycol;
d) emulsifiers such as cetyl dimethicone copolyol, ceteth-20, glyceryl stearate, blend of hydrogenated castor oil and citrate, cetyl alcohol;
d) emollients such as butylene glycol, glycerin, panthenol, propylene glycol.

The efficacy of high surface area zinc oxide as an odour absorbing agent has been demonstrated by *in vivo* and *in vitro* trials. The suitability of high surface area zinc oxide for the purposes stated herein has been demonstrated by trials of standard control formulations against those formulations containing high surface area zinc oxide.

The invention is further disclosed by way of the following, non limiting examples.

Unless otherwise stated, the zinc oxide particles used in these formulations have an average surface area of 90m²/g and an average diameter of 10.47µm.

### Example 1 - Foot Powder + High Surface Area Zinc Oxide

| Ingredient | % |
|---|---|
| Sanitised talc | 47.46 |
| High Surface Area Zinc Oxide | 5.00 |
| Zea Mays | 47.46 |
| Silica | 0.08 |

Silica and half the sanitised talc were sifted through a 40 mesh sieve into a bowl containing the high surface area zinc oxide, then mixed until thoroughly dispersed.

The remaining sanitised talc was sifted into a Simon Solitec Mixer via a 20 mesh sieve. To this was added the high surface area zinc oxide mixture, via a 40 mesh sieve. The Zea Mays was added and the mixture stirred for 10 minutes. A fifth of the mixture was then removed and sifted back into the mixer via a 20 mesh sieve. The mixture was then stirred for a further 20 minutes.

### Example 2 - Foot Powder + High Surface Area Zinc Oxide

| Ingredient | % |
|---|---|
| Sanitised Talc | 47.5 |
| Zea Mays | 47.5 |
| High surface area zinc oxide | 5.0 |

Ingredients were sieved through a 40 mesh sieve and then mixed for 20 minutes.

### Example 3 - Roll-on Deodorant + High Surface Area Zinc Oxide

| Ingredients | % |
|---|---|
| High Surface Area Zinc Oxide | 5.00 |
| Polypropylene glycol - 15 stearyl ether | 4.00 |
| Steareth 21 | 1.50 |
| Steareth 2 | 2.20 |
| Aluminium Chlorohydrate Solution | 30.00 |
| Tetrasodium EDTA | 0.10 |
| Perfume | 0.50 |
| Colour | qs |
| Purified Water | to 100 |

Tetrasodium EDTA was added to purified water, mixed with a homogeniser for 1 minute, then warmed to 70-75°C, until all the solids had dissolved.

Polypropylene glycol -15 stearyl ether, steareth 21, steareth 2 and high surface area zinc oxide were mixed and heated to 70-75°C prior to addition to the tetrasodium EDTA solution. The mixture was homogenised for 5 minutes then stirred slowly whilst cooling to 35°C. Then the aluminium chlorohydrate solution was slowly added to the stirred solution, followed by the perfume. The mixture was stirred until homogenous, then the colour solution was added.

### Example 4 - Absorbent Tablets (Control)

| Ingredients | % |
|---|---|
| Sorbitol | 99.0 |
| Magnesium stearate | 1.00 |

The ingredients were passed through a 30 mesh sieve, blended together in a turbula and then compressed to form the tablets.

### Example 5 - Absorbent Tablets + High Surface Area Zinc Oxide

| Ingredients | % |
|---|---|
| Sorbitol | 98.58 |
| Magnesium stearate | 1.00 |
| High Surface Area Zinc Oxide | 0.42 |

The ingredients were passed through a 30 mesh sieve, blended together in a turbula and then compressed to form the tablets.

### Example 6 - Toothpaste + High Surface Area Zinc Oxide

| Ingredients | % |
|---|---|
| Purified water | 32.23 |
| Sorbitol solution | 45.00 |
| Sodium saccharin | 0.26 |
| Sodium fluoride | 0.24 |
| Hydrated silica | 18.04 |
| Cellulose gum | 0.90 |
| Titanium dioxide | 0.50 |
| Sodium lauryl sulfate | 1.50 |
| Flavour | 0.91 |
| High Surface Area Zinc Oxide | 0.42 |

Sorbitol solution was added to sodium fluoride and sodium saccharin dissolved in water. The solution was placed in the fryma and premixed hydrated silica, cellulose gum, titanium dioxide, sodium lauryl sulfate and high surface area zinc oxide were added. To this mixture was added flavour, then the mixture was stirred under vacuum for 15 minutes.

### Example 7 - Mouthwash + High Surface Area Zinc Oxide

| Ingredients | % |
|---|---|
| High Surface Area Zinc Oxide | 0.42 |
| Purified water | 73.72 |
| Bromochlorophene | 0.10 |
| Flavour | 0.30 |
| Sodium lauryl sulfate | 0.50 |
| Hydrated silica | 10.00 |
| Methylparaben | 0.20 |
| Glycerin | 10.00 |
| Aqueous Montmorillonite solution | 3.50 |
| Colour | 0.0005 |
| Sodium fluoride | 0.26 |
| Sodium saccharin | 1.00 |

To stirred water was added a slurry of aqueous montmorillonite solution, high surface area zinc oxide and glycerin. To this mixture was added a solution of sodium saccharin and sodium fluoride in water, followed by colour, methylparaben, then hydrated silica and sodium lauryl sulfate. Bromochlorophene dissolved in flavour, was added to the mixture, which was then made up to volume with water.

### Example 8 - Toothpaste + High Surface Area Zinc Oxide

| Ingredients | Amount (% w/w) |
|---|---|
| Sorbitol solution | 45.00 |
| Sodium saccharin | 0.26 |
| Sodium fluoride | 0.24 |
| Hydrated silica abrasive | 6.94 |
| Hydrated silica thickener | 11.10 |
| Sodium carboxymethyl cellulose | 0.9 |
| Titanium dioxide | 0.5 |
| Sodium lauryl sulfate | 1.5 |
| Flavour | 0.91 |
| Purified water | to 100 |
| Sodium hydroxide | 0.1 |
| High surface area zinc oxide | 0.9 |

Sodium saccharin, sodium fluoride and sodium hydroxide were dissolved in the water. Sorbitol was then added. Hydrated silica abrasive, hydrated silica thickener sodium, carboxymethylcellulose, titanium dioxide, sodium lauryl sulfate, high surface area zinc oxide and flavour were added and the bulk mixed under vacuum until homogeneous.

### Example 9 - Toothpaste + High Surface Area Zinc Oxide

| Ingredients | Amount (% w/w) |
|---|---|
| Sorbitol solution | 42.00 |
| Sodium saccharin | 0.26 |
| Sodium lauryl sulphate needles | 1.00 |
| Sodium carboxymethyl cellulose for toothpaste | 0.90 |
| Purified water | to 100 |
| Polyethylene glycol 1500 | 1.38 |
| Sodium olefin sulphonate | 2.00 |
| Flavour | 1.00 |
| Toothpaste silica low abrasive | 5.55 |
| Citric acid monohydrate | 0.50 |
| Sodium fluoride | 0.32 |
| Xylitol C | 3.00 |
| Sodium citrate | 2.50 |
| Sodium tripolyphosphate | 5.00 |
| Titanium dioxide | 0.50 |
| Cellulose abrasive | 10.00 |
| High Surface Area Zinc Oxide | 0.90 |

Polyethylene glycol 1500, sodium saccharin, sodium tripolyphosphate, sodium fluoride, citric acid and sodium citrate were dissolved in bulk of water. Sorbitol, xylitol were added and mixed until dissolved. Sodium lauryl sulphate was dissolved in approx. 70g of warm water and sodium olefin sulphonate added to form the surfactant phase. Flavour and surfactant phases were added to the aqueous phase and mixed under high shear to emulsify. Hydrated silica abrasive, sodium carboxymethylcellulose, titanium dioxide, cellulose abrasive and high surface area zinc oxide were added and the bulk mixed under vacuum until homogeneous.

### Example 10 - Toothpaste + High Surface Area Zinc Oxide

| Ingredients | Amount (% w/w) |
|---|---|
| Sodium lauryl sulphate needles | 1.00 |
| Sodium olefin sulphonate | 1.00 |
| Polyethylene glycol 1500 | 1.39 |
| Sodium bicarbonate extra fine | 2.00 |
| Hydrated silica abrasive | 8.33 |
| Hydrated silica thickener | 6.94 |
| Sorbitol solution | 45.00 |
| Titanium dioxide | 0.50 |
| Sodium fluoride | 0.24 |
| Sodium saccharin | 0.26 |
| Sodium carboxymethyl cellulose for toothpaste | 0.90 |
| Xylitol CM | 3.00 |
| Triclosan | 0.30 |
| Flavour | 1.00 |
| Purified water | 19.23 |
| High surface area zinc oxide | 0.90 |

Polyethylene glycol 1500, sodium saccharin, sodium fluoride and sodiumbicarbonate dissolved in bulk of water. Sorbitol and xylitol added and mixed until polyethylene glycol 1500, sodium saccharin, sodium fluoride and sodium dissolved. Sodium lauryl sulphate dissolved in approx. 70g of warm water and sodium olefin sulphonate added. Triclosan was dissolved in flavour. Flavour and surfactant phases added to aqueous phase and mixed under high shear to emulsify. Hydrated silica abrasive, hydrated silica thickener, sodium carboxymethylcellulose, titanium dioxide and high surface area zinc oxide were added and the bulk mixed under vacuum until homogeneous.

### Example 11 - Toothpaste + High Surface Area Zinc Oxide

| Ingredients | Amount (% w/w) |
|---|---|
| Purified water | 78.99 |
| Sodium fluoride | 0.05 |
| Cetylpyridinium chloride | 0.05 |
| Monosodium phosphate fine cryst | 0.50 |
| Disodium phosphate | 0.50 |
| Sodium saccharin | 0.03 |
| Sorbitol | 7.69 |
| PEG-40 hydrogenated castor oil | 0.50 |
| Mixed triglyceride | 0.05 |
| Flavour | 0.15 |
| Alcohol 96% | 9.19 |
| Colour | 1.40 |
| High surface area zinc oxide | 0.90 |

Disodium Phosphate, Monosodium Phosphate, Cetyl Pyridinium Chloride, Sodium Fluoride, Sodium Saccharin, High Surface Area Zinc Oxide and the Colours were dissolved in Water, before adding Sorbitol. Mixed Triglyceride, Flavour, PEG 40 Hydrogenated Castor Oil and Ethanol were mixed until homogeneous, and then added to aqueous phase.

### Example 12 - Stick Deodorant + High Surface Area Zinc Oxide

| Ingredients | Amount (% w/w) |
|---|---|
| Stearyl alcohol vegetable grade | 13.13 |
| Hydrogenated castor oil | 5.05 |
| Cyclomethicone | 50.50 |
| Aluminium chlorohydrate | 20.20 |
| Silica thickener | 1.01 |
| Sterilised Mistron Galaxy talc | 5.59 |
| Mixture of glyceryl stearate and PEG-100 stearate | 1.01 |
| Butylated hydroxy toluene | 0.01 |
| Oily herbal extracts | 0.20 |
| Perfume | 0.50 |
| High surface area zinc oxide | 0.50 |

The stearate mixture, castor oil and stearyl alcohol were melted together. In a separate container the aluminium chlorohydrate and silica thickener were added to the cyclomethicone using a Silverson high shear mixer. The two mixtures were then mixed together and heated to 75-80°C. The talc and butylated hydroxy toluene were added to the resultant mixture whilst it was being stirred. The mixture was stirred with a Silverson high shear mixer to ensure it was homogenous and then cooled to 75°C whilst being stirred. The herbal extract and perfume were added and the mixture was compressed. Finally the composition was poured into suitable packaging and allowed to cool to ambient temperature to form a deodorant with moisturising properties.

A small study was conducted to demonstrate the effect of high surface area zinc oxide *in vitro.*

### IN VITRO EXPERIMENT 1

### METHOD

Seven volunteers gave samples of saliva in the morning before eating or brushing their teeth. 1.3g saliva were measured out into two jars for each sample, and duplicates were made where possible. One jar had the saliva only and one jar had 0.1g of the high surface area zinc oxide in the saliva. These samples were put in a water bath at 37°C for three hours to putrefy with clingfilm around the lip of the jar to provide a head space for the volatile sulfur compounds to build up in. These volatiles were measured in parts per billion (ppb) using a halimeter.

The final concentration of the high surface area zinc oxide in the saliva was 4.8%. The results are shown in Table 1

**TABLE 1**

| SALIVA COMPARED TO SALIVA WITH HIGH SURFACE AREA ZINC OXIDE | | | |
|---|---|---|---|
| | **Halimeter readings (in ppb)** | | |
| **Sample No.** | **Saliva** | **Saliva & High Surface Area ZnO** | **Overall Higher Halimeter Readings** |
| S1 | 76.00 | 143.00 | High surface area ZnO |
| S2 | 1999.00 | 136.00 | Saliva |
| S3 | 1999.00 | 80.00 | Saliva |
| 1S4 | 1999.00 | 64.00 | Saliva |
| 2S4 | 1999.00 | 138.00 | Saliva |
| S5 | 1999.00 | 90.00 | Saliva |
| 1S6 | 1999.00 | 118.00 | Saliva |
| 2S6 | 60.00 | 57.00 | Saliva |
| S7 | 1999.00 | 134.00 | Saliva |

The higher the halimeter reading the more volatile sulphur compounds present. (nb. 1999 is the maximum scale reading for the halimeter).

These results show that the high surface area zinc oxide helps to reduce volatile sulphur compounds *in vitro.*

### IN VIVO EXPERIMENT 1

### METHOD

A small study was carried out to investigate the effect of high surface area zinc oxide *in vivo.*

The high surface area zinc oxide was formulated into a tablet (as in Example 5). This contained a third of the recommended daily allowance of zinc (5 mg/tablet). Control tablets were also formulated containing no high surface area zinc oxide (as in Example 4).

Eleven volunteers had samples of saliva taken after a spicy meal. The volunteers then took a tablet each (control or test) and samples of saliva were obtained an hour later. These samples were processed as for the *in vitro* experiment 1, but no high surface area zinc oxide was added to the jars. The saliva samples were left to putrefy for four hours then halimeter readings were taken. The volatile sulphur compounds present in each sample were then measured in ppb using a halimeter. The process was then repeated two days later, with volunteers receiving the other tablet (control or test) to that which they took previously.

### RESULTS

The results are shown in Table 2.

They showed a reduction in the amount of volatiles in 10 out of the 11 samples from volunteers who had taken high surface area zinc oxide tablet. The control samples showed a 5/11 decrease in the amount of volatiles present.

Chewing a tablet containing high surface area zinc oxide was found to significantly decrease the levels of volatile sulfur compounds found in saliva.

**TABLE 2**

| ANALYSIS OF RESULTS FOR SALIVA PUTRIFICATION READINGS | | | | | | |
|---|---|---|---|---|---|---|
| Sample No. No. | Amount of Volatiles Present in Saliva Before Taking High Surface Area ZnO Tablet (ppb) | Amount of Volatiles Present in Saliva After Taking High Surface Area ZnO Tablet (ppb) | Difference Before-After Taking High Surface Area ZnO Tablet (ppb) | Amount of Volatiles Present in Saliva Before Taking Control Tablet (ppb) | Amount of Volatiles Present in Saliva After Taking Control Tablet (ppb) | Difference Before-After Taking Control Tablet (ppb) |
| 1 | 119.00 | 91.00 | -28.00 | 144.00 | 128.00 | -16.00 |
| 2 | 135.00 | 133.00 | -2.00 | 126.00 | 105.00 | -21.00 |
| 3 | 183.00 | 449.00 | 266.00 | 139.00 | 100.00 | -39.00 |
| 4 | 148.00 | 124.00 | -24.00 | 126.00 | 195.00 | 69.00 |
| 5 | 140.00 | 103.00 | -37.00 | 138.00 | 138.00 | 0.00 |
| 6 | 128.00 | 120.00 | -8.00 | 147.00 | 116.00 | -31.00 |
| 7 | 196.00 | 115.00 | -81.00 | 144.00 | 148.00 | 4.00 |
| 8 | 138.00 | 125.00 | -13.00 | 123.00 | 127.00 | 4.00 |
| 9 | 115.00 | 91.00 | -24.00 | 136.00 | 128.00 | -8.00 |
| 10 | 134.00 | 143.00 | 9.00 | 124.00 | 137.00 | 13.00 |
| 11 | 117.00 | 148.00 | -31.00 | 138.00 | 146.00 | 8.00 |

## Claims

1. The use of a high surface area zinc oxide powder having a surface area between 30m²/g and 100m²/g and a particle size between 0.1 and 200µm in diameter and an acceptable diluent or carrier in the preparation of an odour absorbing composition in which said high surface area zinc oxide absorbs the odour.

2. A use as claimed in claim 1 where the surface area of the zinc oxide is between 90 and 100m²/g and the particle size is between 0.1 and 20.5 µm in diameter.

3. A use as claimed in claim 1 where the odour absorbing composition is topically acceptable.

4. A use as claimed in claim 3 where the high surface area zinc oxide is present from 1 to 10% by weight of the total composition.

5. A use as claimed in claim 3 where the high surface area zinc oxide is present from 3 to 8% by weight of the total composition.

6. A use as claimed in claim 3 where the high surface area zinc oxide is present from 4 to 6% by weight of the total composition.

7. A use as claimed in claim 3 where the composition is formulated as a powder, gel or stick.

8. A use as claimed in claim 1 where the odour absorbing composition is orally acceptable.

9. A use as claimed in claim 8 where the high surface area zinc oxide is present from 0.01 to 10% by weight of the total composition.

10. A use as claimed in claim 8 where the high surface area zinc oxide is present from 0.1 to 5% by weight of the total composition.

11. A use as claimed in claim 8 where the high surface area zinc oxide is present from 0.3 to 1% by weight of the total composition.

12. A use as claimed in claim 8 where the composition is a toothpaste, mouthwash or tablet.

13. A use as claimed in claim 1 wherein an unpleasant smelling active is also used in the preparation of the composition and the high surface area zinc oxide is present to absorb odour within the composition.

14. A use as claimed in claim 13 where the high surface area zinc oxide is present from 1 to 10% by weight of the total composition.

15. A use as claimed in claim 1 where the odour absorbing composition is a medicament for the treatment of bad breath or body odour.

## Patentansprüche

1. Verwendung eines Zinkoxidpulvers hohen Oberflächenbereichs mit einem Oberflächenbereich zwischen 30 m²/g und 100 m²/g und einer Teilchengröße zwischen 0,1 und 200 µm im Durchmesser und eines akzeptablen Verdünnungsmittels oder Trägers in der Herstellung einer geruchabsorbierenden Zusammensetzung, bei der das genannte Zinkoxid hohen Oberflächenbereichs den Geruch absorbiert.

2. Verwendung nach Anspruch 1, bei der der Oberflächenbereich des Zinkoxids zwischen 90 und 100 m²/g and die Teilchengröße zwischen 0,1 und 20,5 µm im Durchmesser beträgt.

3. Verwendung nach Anspruch 1, bei der die geruchabsorbierende Zusammensetzung topisch akzeptabel ist.

4. Verwendung nach Anspruch 3, bei der das Zinkoxid hohen Oberflächenbereichs im Anteil von 1 bis 10 Gewichtsprozent der Gesamtzusammensetzung vorhanden ist.

5. Verwendung nach Anspruch 3, bei der das Zinkoxid hohen Oberflächenbereichs im Anteil von 3 bis 8 Gewichtsprozent der Gesamtzusammensetzung vorhanden ist.

6. Verwendung nach Anspruch 3, bei der das Zinkoxid hohen Oberflächenbereichs im Anteil von 4 bis 6 Gewichtsprozent der Gesamtzusammensetzung vorhanden ist.

7. Verwendung nach Anspruch 3, bei der die Zusammensetzung als ein Puder, Gel oder Stift formuliert ist.

8. Verwendung nach Anspruch 1, bei der die geruchabsorbierende Zusammensetzung oral akzeptabel ist.

9. Verwendung nach Anspruch 8, bei der das Zinkoxid hohen Oberflächenbereichs im Anteil von 0,01 bis 10 Gewichtsprozent der Gesamtzusammensetzung vorhanden ist.

10. Verwendung nach Anspruch 8, bei der das Zinkoxid hohen Oberflächenbereichs im Anteil von 0,1 bis 5 Gewichtsprozent der Gesamtzusammensetzung vorhanden ist.

11. Verwendung nach Anspruch 8, bei der das Zinkoxid hohen Oberflächenbereichs im Anteil von 0,3 bis 1 Gewichtsprozent der Gesamtzusammensetzung vorhanden ist.

12. Verwendung nach Anspruch 8, bei der die Zusammensetzung eine Zahncreme, ein Mundwasser oder eine Tablette ist.

13. Verwendung nach Anspruch 1, bei der bei der Herstellung der Zusammensetzung auch ein unangenehm riechender Wirkstoff verwendet wird und das Zinkoxid hohen Oberflächenbereichs zum Absorbieren von Geruch innerhalb der Zusammensetzung vorhanden ist.

14. Verwendung nach Anspruch 13, bei der das Zinkoxid hohen Oberflächenbereichs im Anteil von 1 bis 10 Gewichtsprozent der Gesamtzusammensetzung vorhanden ist.

15. Verwendung nach Anspruch 1, bei der die geruchabsorbierende Zusammensetzung ein Medikament zur Behandlung von Mundgeruch oder Körpergeruch ist.

## Revendications

1. L'utilisation d'une poudre d'oxyde de zinc à grande surface spécifique possédant une surface spécifique qui est située entre 30 m²/g et 100 m²/g et une taille de particules située entre un diamètre de 0,1 et 200 µm ainsi qu'un diluant ou une substance de support acceptable dans la préparation d'une composition d'absorption des odeurs, dans laquelle ledit oxyde de zinc à grande surface spécifique absorbe l'odeur.

2. Utilisation, selon la revendication 1, dans laquelle la surface spécifique de l'oxyde de zinc est située entre 90 et 100 m²/g et la taille des particules est située entre un diamètre de 0,1 et 20,5 µm.

3. Utilisation, selon la revendication 1, dans laquelle la composition d'absorption des odeurs est acceptable sur le plan topique.

4. Utilisation, selon la revendication 3, dans laquelle l'oxyde de zinc à grande surface spécifique est présent à hauteur de 1 à 10% par poids de la composition totale.

5. Utilisation, selon la revendication 3, dans laquelle l'oxyde de zinc à grande surface spécifique est présent à hauteur de 3 à 8% par poids de la composition totale.

6. Utilisation, selon la revendication 3, dans laquelle l'oxyde de zinc à grande surface spécifique est présent à hauteur de 4 à 6% par poids de la composition totale.

7. Utilisation, selon la revendication 3, dans laquelle la composition est formulée en tant que poudre, gel ou bâtonnet.

8. Utilisation, selon la revendication 1, dans laquelle la composition d'absorption des odeurs est acceptable sur le plan oral.

9. Utilisation, selon la revendication 8, dans laquelle l'oxyde de zinc à grande surface spécifique est présent à hauteur de 0,01 à 10% par poids de la composition totale.

10. Utilisation, selon la revendication 8, dans laquelle l'oxyde de zinc à grande surface spécifique est présent à hauteur de 0,1 à 5% par poids de la composition totale.

11. Utilisation, selon la revendication 8, dans laquelle l'oxyde de zinc à grande surface spécifique est présent à hauteur de 0,3 à 1% par poids de la composition totale.

12. Utilisation, selon la revendication 8, dans laquelle la composition est un dentifrice, un bain de bouche ou un comprimé.

13. Utilisation, selon la revendication 1, dans laquelle un agent actif à odeur désagréable est également utilisé dans la préparation de la composition et l'oxyde de zinc à grande surface spécifique est présent pour absorber l'odeur dans la composition.

14. Utilisation, selon la revendication 13, dans laquelle l'oxyde de zinc à grande surface spécifique est présent à hauteur de 1 à 10% par poids de la composition totale.

15. Utilisation, selon la revendication 1, dans laquelle la composition d'absorption des odeurs est un médicament destiné au traitement de la mauvaise haleine ou de l'odeur corporelle.
